(19) Europäisches Patentamt
European Patent Office
Office européen des brevets

(11) Veröffentlichungsnummer : **0 084 149**
**B1**

(12) # EUROPÄISCHE PATENTSCHRIFT

(45) Veröffentlichungstag der Patentschrift : **05.03.86**

(51) Int. Cl.⁴ : **C 07 C127/19**, A 01 N 47/30

(21) Anmeldenummer : **82111820.5**

(22) Anmeldetag : **20.12.82**

(54) **Phenoxyphenylharnstoffe, Verfahren zu ihrer Herstellung und ihre Verwendung zur Bekämpfung unerwünschten Pflanzenwuchses.**

(30) Priorität : **20.01.82 DE 3201525**

(43) Veröffentlichungstag der Anmeldung : **27.07.83 Patentblatt 83/30**

(45) Bekanntmachung des Hinweises auf die Patenterteilung : **05.03.86 Patentblatt 86/10**

(84) Benannte Vertragsstaaten : **AT BE DE FR GB IT NL**

(56) Entgegenhaltungen :
**DE-A- 2 024 249**
**DE-A- 2 413 722**
**DE-B- 1 142 251**

(73) Patentinhaber : **BASF Aktiengesellschaft**
**Carl-Bosch-Strasse 38**
**D-6700 Ludwigshafen (DE)**

(72) Erfinder : **Becker, Rainer, Dr.**
**Im Haseneck 22**
**D-6702 Bad Duerkheim (DE)**
Erfinder : **Varwig, Juergen, Dr.**
**Am Goetzenberg 1**
**D-6900 Heidelberg (DE)**
Erfinder : **Wuerzer, Bruno, Dr.**
**Ruedigerstrasse 13**
**D-6701 Otterstadt (DE)**

**EP 0 084 149 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Phenoxyphenylharnstoffe, Verfahren zu ihrer Herstellung, Herbizide, die diese Verbindungen als Wirkstoffe enthalten, sowie ein Verfahren zur Bekämpfung unerwünschten Pflanzenwuchses mit diesen Wirkstoffen.

Phenoxyphenylharnstoffe mit herbiziden Eigenschaften sind bekannt (DE-AS 11 42 251). So wird z. B. N-[4-(4'-Chlorphenoxy)-phenyl]-N',N'-dimethyl-harnstoff zur Unkrautbekämpfung in Sojabohnen, Sellerie, Erdbeeren und Karotten verwendet (Farm Chemicals Handbook 1981, C 327 Meister Publishing Co., Willough by Ohio). Außerdem is bekannt, daß 4-(4-Alkoxy-phenoxy)-phenyl-harnstoffe als selektive Herbizide Verwendung finden können (DE-OS 19 18 114).

Es wurde gefunden, daß Phenoxyphenylharnstoffe der Formel

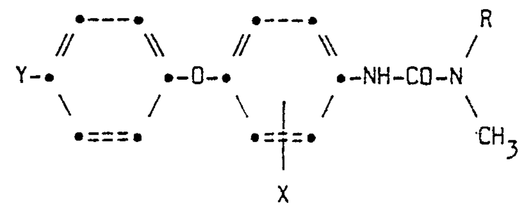

(I)

in der

R Wasserstoff, Methyl oder Methoxy,

X Wasserstoff, Halogen oder Trifluormethyl, und

Y $C_1$-$C_3$-Halogenalkoxy bedeuten,

eine gute herbizide Wirkung und für eine Reihe von Kulturpflanzen eine gute Verträglichkeit haben.

In der Formel I bedeutet X Wasserstoff, Trifluormethyl oder Halogen, wie Fluor, Chlor, Brom, insbesondere Chlor ; Y bedeutet einen $C_1$-$C_3$-Halogenalkoxyrest, wie Difluormethoxy, Trifluormethoxy, 1,1,2,2-Tetrafluorethoxy, 1,1,2-Trifluor-2-chlor-ethoxy, 1,1,2-Trifluor-2-brom-ethoxy, 1,1,2,3,3,3-Hexafluorn-propoxy.

Bevorzugte Phenoxyphenylharnstoffe der Formel I sind solche, bei denen R Methoxy oder Methyl bedeutet.

Man erhält die Phenoxyphenylharnstoffe der Formel I durch Umsetzung von 4-(4-Halogenalkoxy-phenoxy)-anilinderivaten der Formel

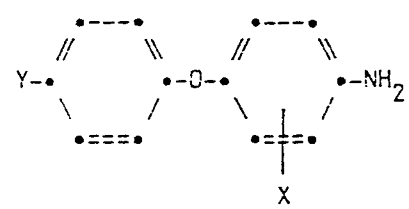

(II)

in der X und Y die obengenannten Bedeutungen haben, mit einem Carbaminsäurechlorid der Formel $R—N(CH_3)—CO—Cl$, wobei R die obengenannten Bedeutungen hat, oder mit Methylisocyanat bei Temperaturen zwischen 0 und 50 °C in einem inerten Lösungsmittel. Geeignete Lösungsmittel sind beispielsweise Toluol, Tetrahydrofuran, Pyridin, Acetonitril. Bei Verwendung der Säurechloride wird zweckmäßigerweise eine Hilfsbase, wie Triäthylamin oder Kaliumcarbonat, zugegeben.

Weiterhin werden die Phenoxyphenylharnstoffe der Formel I durch Phosgenierung der Aniline der Formel II in an sich bekannter Weise und durch Umsetzung der so erhaltenen Phenoxyphenylisocyanate mit Aminen der Formel $NH(CH_3)R$ erhalten. R hat dabei die oben angegebenen Bedeutungen.

Die 4-(4-Halogenalkoxy-phenoxy)-anilin-derivate der Formel II können in an sich üblicher Weise auf folgendem Wege erhalten werden :

Man setzt Hydrochinon mit 4-Halogennitrobenzolderivaten der Formel

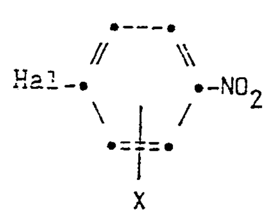

in der X die obengenannten Bedeutungen hat und Hal für Halogen steht, in Gegenwart einer Base und eines Lösungsmittels, wie Dimethylsulfoxid, bei einer Temperatur von 30 bis 80 °C zu 4-(4-Hydroxyphenoxy)-nitrobenzolderivaten der Formel

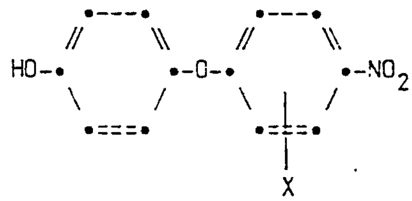

(III)

in der X die obengenannten Bedeutung hat, um. Durch Umsetzung dieser Nitroverbindungen mit Halogenalkyl- oder Halogenalkenylverbindungen der Formeln A-Hal, wobei A einen halogenierten Alkyl- oder Alkenylrest und Hal Halogen bedeutet, in Gegenwart einer Base und einem Lösungsmittel, wie Dioxan, bei einer Temperatur von 30 bis 80 °C und anschließende Reduktion der Nitroverbindung zum Amin, beispielsweise durch katalytische Hydrierung mit Raney-Nickel oder mit Palladium auf Kohle oder mit Zinn(II)-chlorid oder Eisenpulver, erhält man die Verbindungen der Formel II.

Als Halogenalkyl- bzw. Halogenalkenylverbindungen der Formel A-Hal kommen beispielsweise Chlordifluormethan, Chlortrifluorethylen, Bromtrifluorethylen, Hexafluorpropen oder Tetrafluorethylen in Betracht.

Die 4-(4-Halogenalkoxy-phenoxy)-anilinderivate der Formel II können auch durch Umsetzung von 4-Halogenalkoxy-phenolen der Formel

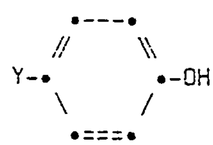

mit 4-Halogen-nitrobenzolderivaten der Formel

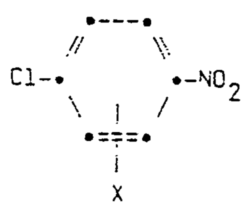

und anschließende Reduktion der 4-(4-Halogenoxy-phenoxy)-nitrobenzolderivate der Formel

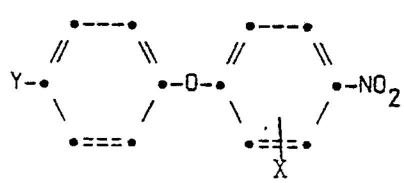

erhalten werden.

Die folgenden Beispiele erläutern die Herstellung der Phenoxyphenyl-harnstoffe der Formel I. Gewichtsteile verhalten sich zu Volumenteilen wie Kilogramm zu Liter.

## Beispiel 1

99 Gewichtsteile Hydrochinon werden in 1 000 Volumenteilen Dimethylsulfoxid gelöst und mit 33 Gewichtsteilen Calcium-hydroxid versetzt. 101 Gewichtsteile 2-Chlor-5-nitrobenzotrifluorid wurden zugefügt und die Mischung wurde bei 50 bis 60 °C 10 Stunden gerührt. Anschließend wurde in kalte, verdünnte Salzsäure eingerührt und abgesaugt. Es wurden 133,4 g 4-Hydroxy-2′-trifluormethyl-4′-nitrodiphenylether vom Schmelzpunkt 118-120 °C erhalten.

$C_{13}H_8NO_4F_3$/M = 299

Ber. : C 52,19 % H 2,70 % N 4,68 % F 19,05 %

Gef. : C 52,2 % H 2,8 % N 4,5 % F 18,2 %

157 Gewichtsteile 4-Hydroxy-2′-trifluormethyl-4′-nitrodiphenylether werden in 250 Volumenteilen

Dioxan gelöst und mit 105 Gewichtsteilen Natriumhydroxid in 250 Volumenteilen Wasser versetzt. Bei einer Temperatur von 60-65 °C wird Chlordifluormethan eingeleitet. Nach 4 Stunden ist die Reaktion beendet (Dünnschichtkontrolle). Es wird von etwas ungelösten Rückstand abfiltriert, dann wird in Methylenchlorid aufgenommen und mit Wasser neutral gewaschen.

Nach dem Einengen verbleiben 179 g 4-Difluormethoxy-2'-trifluormethyl-4'-nitrodiphenylether als gelbes Öl.

$C_{14}H_8F_5NO_4$/M = 349

88 Gewichtsteile 4-Difluormethoxy-2'-trifluormethyl-4'-nitrodiphenylether wurden in 97 Volumenteilen Ethanol und 118 Volumenteil konz. Salzsäure suspendiert und bei 40 bis 50 °C portionsweise mit 204 g Zinn(II)-chlorid versetzt. Es wurde zwei Stunden bei 60 °C nachgerührt und dann in 660 g Eis und 430 Volumenteilen 50 %ige Natronlauge eingerührt, mit Methylenchlorid extrahiert und die Methylenchloridphase eingeengt. Das erhaltene Öl wurde mit wäßriger 10 %iger Salzsäure in das Hydrochlorid überführt. Dabei wurden 58 g 4-Difluormethoxy-2'-trifluormethyl-4'-amino-diphenyletherhydrochlorid erhalten.

$C_{14}H_{11}NClF_5O_2$/M=395

Ber. : C 47,28 % H 3,12 % N 3,94 % Cl 9,97 % F 26,71 %

Gef. : C 47,1 % H 3,3 % N 4,0 % Cl 9,4 % F 25,9 %

10,7 Gewichtsteile 4-Difluormethoxy-2'-trifluormethyl-4'-aminodiphenyletherhydrochlorid werden in 150 Volumenteilen Pyridin aufgenommen und bei 10 °C tropfenweise mit 3,7 Gewichtsteilen Methoxymethylcarbaminsäurechlorid versetzt. Nach 10-stündigem Rühren wird in kalte Salzsäure eingerührt und der ausgefallene N-[4-(4-Difluormethoxy-phenoxy)-3-trifluormethylphenyl]-N'-methyl-N'-methoxyharnstoff abgesaugt. Ausbeute : 11,3 Geiwchtsteile (Schmelzpunkt 65-68 °C) (Wirkstoff Nr. 8).

$C_{17}H_{15}N_2O_4F_5$ (M = 391)

Beispiel 2

19,4 Gewichtsteile 4-(1,1,2-Trifluor-2-chlorethoxy)-2'-chlor-4'-amino-diphenylether wurden in 100 Volumenteilen Pyridin gelöst und tropfenweise mit 5,6 Gewichtsteilen Dimethylcarbaminsäurechlorid versetzt. Nach 20 Stunden wurde in Eiswasser/Salzsäure eingerührt und abgesaugt. Es wurden 18,9 g N-[4-[4-(1,1,2-Trifluor-2-chlorethoxy)-phenoxy]-3-chlor]-phenyl-N',N'-dimethylharnstoff vom Schmelzpunkt 116 bis 118 °C erhalten. (Wirkstoff Nr. 13).

$C_{17}H_{15}N_2Cl_2F_3O_3$/M = 423

Ber. : C 48,25 % H 3,57 % N 6,62 % F 13,47 %

Gef. : C 47,9 % H 3,7 % N 6,0 % F 13,4 %

Die folgenden Phenoxyphenylharnstoffe der Formel I wurden auf analogem Wege erhalten :

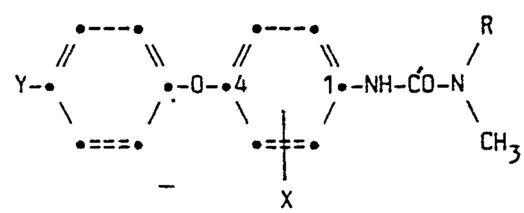

| Nr. | X | Y | R | Fp. [°C] |
|---|---|---|---|---|
| 1 | H | $OCHF_2$ | H | 157-159 |
| 3 | 3-Cl | $OCHF_2$ | $CH_3$ | 125-127 |
| 6 | 3-Cl | $OCF_2CHFCl$ | $OCH_3$ | zähes Öl |
| 8 | $3-CF_3$ | $OCHF_2$ | $OCH_3$ | 65-68 |
| 10 | H | $OCF_2CHFCl$ | $CH_3$ | 92-94 |
| 13 | 3-Cl | $OCF_2CHFCl$ | $CH_3$ | 116-118 |
| 15 | 3-Cl | $OCHF_2$ | H | 122-123 |
| 18 | H | $OCHF_2$ | $OCH_3$ | 64-66 |
| 19 | 3-Cl | $OCHF_2$ | $OCH_3$ | 75-77 |
| 23 | $3-CF_3$ | $OCHF_2$ | $CH_3$ | zähes Öl |
| 25 | H | $OCF_2CHFCl$ | $OCH_3$ | 55-58 |
| 27 | H | $OCHF_2$ | $CH_3$ | 93-94 |

(Fortsetzung)

| Nr. | X | Y | R | Fp. [°C] |
|---|---|---|---|---|
| 29 | H | $OCF_2CHFCl$ | H | 135-136 |
| 31 | 3-Cl | $OCF_2CHFCl$ | H | 121-122 |
| 42 | $3-CF_3$ | $OCF_3$ | $CH_3$ | 75-78 |
| 43 | $3-CF_3$ | $OCF_3$ | $OCH_3$ | 98-100 |

Die folgenden Phenoxyphenylharnstoffe der Formel I können auf analogem Wege erhalten werden :

| Nr. | X | Y | R | Fp [°C] |
|---|---|---|---|---|
| 2 | $2-CF_3$ | $OCHF_2$ | $OCH_3$ | |
| 4 | 3-F | $OCHF_2$ | $CH_3$ | |
| 5 | 3-Cl | $OCF_2CHFBr$ | $CH_3$ | |
| 7 | H | $OCF_2CHFCF_3$ | H | |
| 9 | 3-Cl | $OCF_2CHFBr$ | $OCH_3$ | |
| 11 | H | $OCF_2CHFCF_3$ | $OCH_3$ | |
| 12 | 3-Cl | $OCF_2CHF_2$ | $OCH_3$ | |
| 14 | $3-CF_3$ | $OCF_2CHFBr$ | $OCH_3$ | |
| 16 | 3-Cl | $OCF_2CHFCF_3$ | $OCH_3$ | |
| 17 | $3-CF_3$ | $OCF_2CHFCF_3$ | $OCH_3$ | |
| 20 | $3-CF_3$ | $OCF_2CHFCF_3$ | $CH_3$ | |
| 21 | 3-Cl | $OCF_2CHFBr$ | H | |
| 22 | $3-CF_3$ | $OCF_2CHF_2$ | $OCH_3$ | |
| 24 | $3-CF_3$ | $OCF_2CHFBr$ | $CH_3$ | |
| 26 | 3-Cl | $OCF_2CHFCF_3$ | $CH_3$ | |
| 28 | 3-Cl | $OCF_2CHF_2$ | $CH_3$ | |
| 30 | 3-Cl | $OCF_2CHFCF_3$ | H | |
| 32 | $3-CF_3$ | $OCF_2CHF_2$ | $CH_3$ | |
| 33 | 3-Cl | $OCF_2CHF_2$ | H | |
| 34 | $2-CF_3$ | $OCHF_2$ | $CH_3$ | |
| 35 | $2-CF_3$ | $OCHF_2$ | H | |
| 36 | $2-CF_3$ | $OCHF_2$ | $OCH_3$ | |
| 37 | 3-Br | $OCHF_2$ | $OCH_3$ | |
| 38 | $3-CF_3$ | $OCF_3$ | H | |
| 39 | H | $OCF_3$ | $CH_3$ | |
| 40 | 3-Cl | $OCF_3$ | $CH_3$ | |
| 41 | H | $OCF_3$ | $OCH_3$ | |
| 44 | 3-Cl | $OCF_3$ | $OCH_3$ | |

Die Phenoxyphenylharnstoffe der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende konzentrate hergestellt werden, die zur Verdünnung mit Wasser geeignet sind.

Als oberflächenaktive Stoffe kommen Alkali-, Erdalikali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetat, Sorbitester, Lignin, Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff,

Beispiele für Formulierungen sind :

I. Man vermischt 90 Gewichtsteile der Verbindung Nr. 5 mit 10 Gewichtsteilen N-Methyl- pyrrolidon und erhält eine Lösung, die zur Anwendung in Form kleinster Tropfen geeignet ist.

II. 20 Gewichtsteile der Verbindung Nr. 13 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecyl-benzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 19 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile der Verbindung Nr. 50 werden in einer Mischung gelost die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile der Verbindung Nr. 64 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigen Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile der Verbindung Nr. 36 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile der Verbindung Nr. 46 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 23 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder bei Nachauflaufanwendung erfolgen. Vorzugsweise werden die neuen Wirkstoffe nach dem Auflaufen oder während des Auflaufens der unerwünschten Pflanzen, sowohl auf Kulturflächen als auch auf unbebautem Land, ausgebracht. Sind die Wirkstoffe für die Kulturpflanze weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bodenart, Jahreszeit, Zielpflanzen und Wachstumsstadium 0,05 bis 5 kg/ha und mehr, vorzugsweise 0,1 bis 3,0 kg/ha, wobei sich die höheren Dosen besonders zur totalen Bekämpfung von Vegetationen eignen.

Die herbizide Wirkung von Verbindungen der Formel I wird durch Gewächshausversuche gezeigt :

Als Kulturgefäße dienten Plastikblumentöpfe mit 300 $cm^3$ Inhalt und lehmigem Sand mit etwa 1,5 % Humus als Substrat. Die Samen der Testpflanzen wurden nach Arten getrennt flach eingesät. Unmittelbar danach erfolgt bei Vorauflaufbehandlung das Aufbringen der Wirkstoffe bzw. der sie enthaltenden herbiziden Mittel auf die Erdoberfläche. Sie wurden hierzu in Wasser als Verteilungsmittel suspendiert oder emulgiert und mittels fein verteilender Düsen gespritzt. Die Aufwandmengen betrugen 3,0 kg Wirkstoff/ha. Nach dem Aufbringen der Mittel beregnete man die Gefäße leicht, um Keimung und Wachstum in Gang zu bringen. Danach deckte man die Gefäße mit durchsichtigen Plastikhauben ab, bis die Pflanzen angewachsen sind. Diese Abdeckung bewirkte ein gleichmäßiges Keimen der Testpflanzen, sofern dies nicht durch die Wirkstoffe beeinträchtigt wurde.

Für die Nachauflaufbehandlung zog man die Testpflanzen je nach Wuchsform erst bis zu einer Wuchshöhe von 3 bis 15 cm an und behandelte sie danach. Die für die Nachauflaufanwendung eingesetzten Soja- und Klettenlabkrautpflanzen zog man in einem mit Torfmull (peat) angereicherten Substrat an, um ein günstigeres Wachstum zu gewährleisten. Zur Nachauflaufbehandlung wurden entweder direkt gesäte und in den gleichen Gefäßen aufgewachsene Pflanzen ausgewählt, oder aber sie wurden erst als Keimpflanzen getrennt angezogen und einige Tage vor der Behandlung in die Versuchsgefäße verpflanzt. Die Aufwandmengen für die Nachauflaufbehandlung variierten je nach Wirkstoff. Sie betrugen 0,125, 0,25, 0,5, 1,0 bzw. 3,0 kg Wirkstoff/ha. Eine Abdeckung unterblieb bei der Nachauflaufbehandlung.

Die Versuchsgefäße wurden im Gewächshaus aufgestellt, wobei für wärmeliebende Arten wärmere Bereiche (20 bis 30 °C) und für solche gemäßigter Klimate 15 bis 25 °C bevorzugt wurden. Die Versuchsperiode erstreckte sich über 2 bis 4 Wochen. Während dieser Zeit wurden die Pflanzen gepflegt, und ihre Reaktion auf die einzelnen Behandlungen wurde ausgewertet. Bewertet wurde nach einer Skala von 0 bis 100. Dabei bedeutet 0 keine Schädigung oder normaler Auflauf und 100 kein Aufgang der Pflanzen bzw. völlige Zerstörung zumindest der oberirdischen Teile.

Die Testpflanzen setzten sich aus folgenden Arten zusammen :

Amaranthus retroflexus (zurückgekrümmter Fuchsschwanz), Arachys hypogaea (Erdnüsse), Avena sativa (Hafer), Cassia tora, Centaurea cyanus (Kornblume), Chenopodium album (Weißer Gänsefuß), Euphorbia geniculata (Südamerikan. Wolfsmilchart), Galium aparine (Kletten-Labkraut), Glycine max. (Soja), Ipomoea spp. (Prunkwinde), Lamium purpureum (Rote Taubnessel), Lolium multiflorum (Ital. Raygras), Mentha piperita (Pfefferminze), Sinapis alba (Weißer Senf), Solanum nigrum (Schwarzer Nachtschatten) und Triticum aestivum (Weizen).

Herbizide Vergleichsmittel sind N-[4-(4-Chlorphenoxy)-phenyl]-N',N'-dimethylharnstoff (A) und N-[4-(4-Methoxyphenoxy)-3-chlor-phenyl]-N'-methoxy-N'-methyl-harnstoff (B).

Bei Vorauflaufanwendung im Gewächshaus zeigten die Verbindungen Nr. 1, 3, 8, 10 und 25 bei Aufwandmengen von 3,0 kg Wirkstoff/ha eine gute herbizide Aktivität, insbesondere gegen weißen Senf, ohne die Getreideart Hafer zu schädigen.

Die Verbindungen Nr. 1, 3 und 6 haben eine beachtliche herbizide Wirkung gegen eine Reihe von Beispielspflanzen bei Nachauflaufanwendung von 3,0 kg Wirkstoff/ha. Die Verbindungen Nr. 10, 13, 19, 25 und 27 bekämpfen mit 0,5 bzw. 1,0 kg Wirkstoff/ha im Nachauflaufverfahren breitblättrige unerwünschte Pflanzen in Weizen.

Die Verbindung Nr. 8 und 23 sind mit 0,125 kg/ha bei Nachauflaufbehandlung bei gleichzeitig besserer herbizider Aktivität wie das Vergleichsmittel A selektiv für die Kulturpflanzen, während die

Verbindung Nr. 19 bei Nachauflaufanwendung von 0,25 und 0,5 kg Wirkstoff/ha eine höhere Aktivität als das Vergleichsmittel B hat.

In Anbetracht der Verträglichkeit und der Vielseitigkeit der Applikationsmethoden, können die erfindungsgemäßen Verbindungen noch in einer weiteren Zahl von Kulturpflanzen zur Beseitigung unerwünschter Pflanzen eingesetzt werden.

In Betracht kommen beispielsweise folgende Kulturen :

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Camellia sinensis | Teestrauch |
| Carthamus tinctorius | Saflor-Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Linum usitatissimum | Faserlein |
| Malus spp. | Apfel |
| Manihot esculenta | Maniok |
| Medicago sativa | Luzerne |
| Mentha piperita | Pfefferminze |
| Musa spp. | Obst- u. Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |

| Botanischer Name | Deutscher Name |
|---|---|
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Spinacia oleracea | Spinat |
| Theobroma cacao | Kakaobaum |
| Triticum aestivum | Weizen |
| Vaccinium carymbosum | Kulturheidelbeere |
| Vaccinium vitis-idaea | Preißelbeere |

Zur Verbreiterung des Wirkungsspektrums und zur Erzielung synergistischer Effekte können die erfindungsgemäßen Verbindungen mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

**Patentansprüche**

1. Phenoxyphenylharnstoffe der Formel

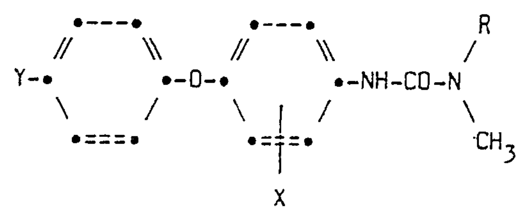

(I)

in der

R Wasserstoff, Methyl oder Methoxy,

X Wasserstoff, Halogen oder Trifluormethyl und

Y $C_1$-$C_3$-Halogenalkoxy bedeuten.

2. Phenoxyphenylharnstoffe der Formel I gemäß Anspruch 1, dadurch gekennzeichnet, daß R Methyl oder Methoxy bedeutet.

3. N-[4-(4-Difluormethoxy-phenoxy)-3-trifluormethylphenyl]-N'-methyl-N'-methoxy-harnstoff.

4. N-[4-(4-Difluormethoxy-phenoxy)-3-chlor-phenyl]-N'-methyl-N'-methoxy-harnstoff.

5. N-[4-(4-Difluormethoxy-phenoxy)-3-trifluormethylphenyl]-N',N'-dimethyl-harnstoff.

6. Verfahren zur Herstellung von Phenoxyphenylharnstoffen der Formel

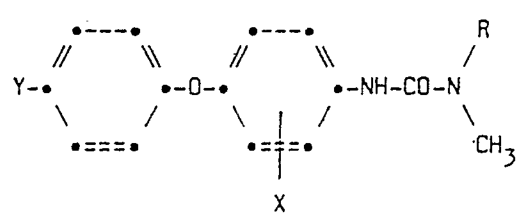

(I)

in der

R Wasserstoff, Methyl oder Methoxy,

X Wasserstoff, Halogen oder Trifluormethyl und

Y $C_1$-$C_3$-Halogenalkoxy bedeuten,

dadurch gekennzeichnet, daß man 4-(4-Halogenalkoxyphenoxy)-anilinderivate der Formel

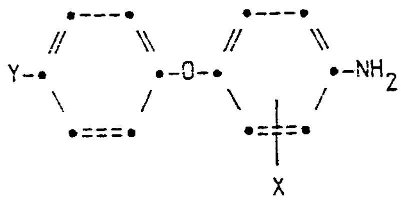

(II)

in der X und Y die obengenannten Bedeutungen haben, in Gegenwart eines inerten Lösungsmittels mit einem Carbaminsäurechlorid der Formel

$$R—N(CH_3)—CO—Cl$$

wobei R die obengenannten Bedeutungen hat, oder mit Methylisocyanat bei einer Temperatur zwischen 0 und 50 °C umsetzt.

7. Herbizid, enthaltend einen Phenoxyphenylharnstoff der Formel

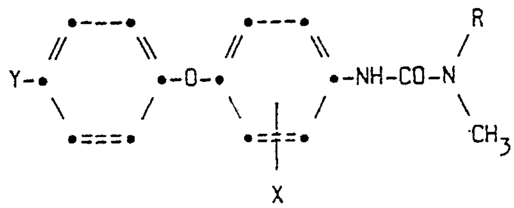

(I)

in der

R Wasserstoff, Methyl oder Methoxy,

X Wasserstoff, Halogen oder Trifluormethyl und

Y $C_1$-$C_3$-Halogenalkoxy bedeuten.

8. Herbizid, enthaltend inerte Zusatzstoffe und einen Phenoxyphenylharnstoff der Formel I gemäß Anspruch 7.

9. Verfahren zur Bekämpfung unerwünschten Pflanzenwuchses, dadurch gekennzeichnet, daß man die unerwünschten Pflanzen oder die von unerwünschtem Pflanzenwuchs freizuhaltende Fläche mit einer herbizid wirksamen Menge eines Phenoxyphenylharnstoffs der Formel I gemäß Anspruch 1 behandelt.

**Claims**

1. A phenoxyphenylurea of the formula

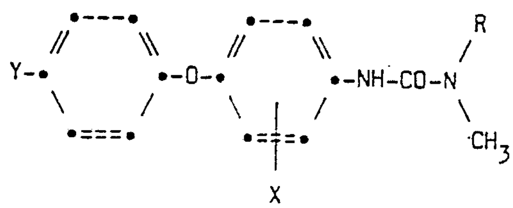

(I)

where

R is hydrogen, methyl or methoxy,

X is hydrogen, halogen or trifluoromethyl, and

Y is $C_1$-$C_3$-haloalkoxy.

2. A phenoxyphenylurea of the formula I as claimed in claim 1, wherein R is methyl or methoxy.

3. N-[4-(4-Difluoromethoxyphenoxy)-3-trifluoromethylphenyl]-N'-methyl-N'-methoxyurea.

4. N-[4-(4-Difluoromethoxyphenoxy)-3-chlorophenyl]-N'-methyl-N'-methoxyurea.

5. N-[4-(4-Difluoromethoxyphenoxy)-3-trifluoromethylphenyl]-N',N'-dimethylurea.

6. A process for the preparation of phenoxyphenylurea of the formula

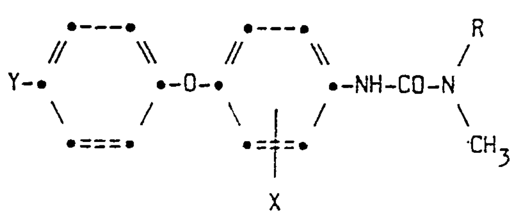

(I)

where

R is hydrogen, methyl or methoxy,

X is hydrogen, halogen or trifluoromethyl, and

Y is $C_1$-$C_3$-haloalkoxy,

wherein a 4-(4-haloalkoxyphenoxy)-aniline derivative of the formula

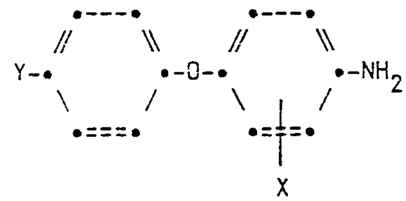

(II)

where X and Y have the above meanings, is reacted with a carbamyl chloride of the formula

$$R—N(CH_3)—CO—Cl,$$

where R has the above meanings, or with methyl isocyanate, at from 0 to 50 °C in an inert solvent.

7. A herbicide containing a phenoxyphenylurea of the formula

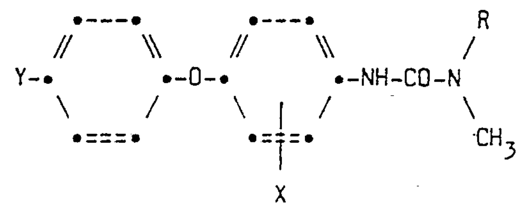

(I)

where

R is hydrogen, methyl or methoxy,

X is hydrogen, halogen or trifluoromethyl, and

Y is $C_1$-$C_3$-haloalkoxy.

8. A herbicide containing inert additives and a phenoxyphenylurea of the formula I as claimed in claim 7.

9. A process for combating the growth of unwanted plants wherein the unwanted plants or the area to be kept free from unwanted plant growth are treated with a herbicidally effective amount of a phenoxyphenylurea of the formula I as claimed in claim 1.

**Revendications**

1. Phénoxyphénylurées de formule

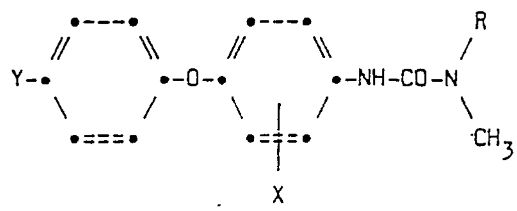

(I)

dans laquelle

R représente hydrogène, méthyle ou méthoxy,

X représente hydrogène, halogène ou trifluorométhyle et

Y représente halogénalcoxy en $C_1$-$C_3$.

2. Phénoxyphénylurées de formule I, selon la revendication 1, caractérisées par le fait que R représente méthyle ou méthoxy.

3. N-[4-(4-difluorométhoxy-phénoxy)-3-trifluorométhylphényl]-N'-méthyl-N'-méthoxy-urée.

4. N-[4-(4-difluorométhoxy-phénoxy)-3-chlorophényl]-N'-méthyl-N'-méthoxy-urée.

5. N-[4-(4-difluorométhoxy-phénoxy)-3-trifluorométhylphényl]-N',N'-diméthyl-urée.

6. Procédé de préparation de phénoxyphénylurées de formule

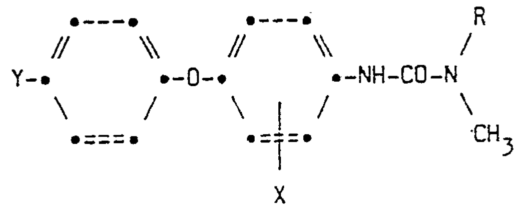

(I)

dans laquelle

R représente hydrogène, méthyle ou méthoxy,

X représente hydrogène, halogène ou trifluorométhyle et

Y représente halogénalcoxy en $C_1$-$C_3$,

caractérisé par le fait que l'on fait réagir des dérivés de 4-(4-halogénalcoxy-phénoxy)-aniline de formule

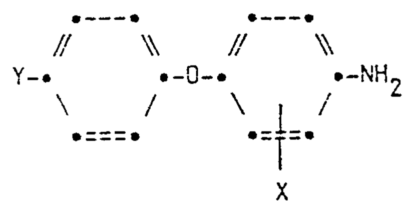

(II)

dans laquelle X et Y ont les significations sus-indiquées, en présence d'un solvant inerte, avec un chlorure d'acide carbaminique de formule

$$R—N(CH_3)CO—Cl$$

où R a les significations sus-indiquées, ou avec de l'isocyanate de méthyle, à une température comprise entre 0 et 50 °C.

7. Herbicide, contenant un phénoxyphénylurée de formule

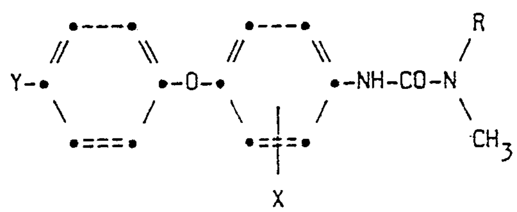

(I)

dans laquelle

R représente hydrogène, méthyle ou méthoxy,

X représente hydrogène, halogène ou trifluorométhyle et

Y représente halogénalcoxy en $C_1$-$C_3$.

8. Herbicide contenant des additifs inertes et un phénoxyphénylurée de formule I selon la revendication 7.

9. Procédé de lutte contre la croissance indésirable de plantes, caractérisé par le fait que l'on traite les plantes indésirables ou les surfaces à libérer de la croissance des plantes indésirables avec une quantité efficace, du point de vue herbicide, d'une phénoxy-phényl-urée de formule I selon la revendication 1.